(19) Europäisches Patentamt
European Patent Office
Office européen des brevets

(11) EP 1 106 249 A1

(12) EUROPEAN PATENT APPLICATION
published in accordance with Art. 158(3) EPC

(43) Date of publication:
**13.06.2001 Bulletin 2001/24**

(21) Application number: **00937189.9**

(22) Date of filing: **08.06.2000**

(51) Int Cl.7: **B01J 31/26**, C07C 2/32, C07C 11/02, C08F 4/80

(86) International application number:
**PCT/JP00/03724**

(87) International publication number:
**WO 00/76659 (21.12.2000 Gazette 2000/51)**

(84) Designated Contracting States:
**AT BE CH CY DE DK ES FI FR GB GR IE IT LI LU MC NL PT SE**
Designated Extension States:
**AL LT LV MK RO SI**

(30) Priority: **11.06.1999 JP 16499999**

(71) Applicant: **IDEMITSU PETROCHEMICAL CO. Ltd.**
**Tokyo 130-0015 (JP)**

(72) Inventors:
- **Sato, Haruhito**
  **Ichihara-shi, Chiba 299-0107 (JP)**
- **Kuramoto, Masahiko**
  **Ichihara-shi, Chiba 299-0107 (JP)**
- **Watanabe, Masami**
  **Ichihara-shi, Chiba 299-0107 (JP)**
- **Tanaka, Shinji**
  **Tokuyama-shi, Yamaguchi 745-0843 (JP)**

(74) Representative:
**Gille Hrabal Struck Neidlein Prop Roos**
**Patentanwälte**
**Brucknerstrasse 20**
**40593 Düsseldorf (DE)**

# (54) CATALYST FOR $g(a)-OLEFIN PRODUCTION AND PROCESS FOR PRODUCING $g(a)-OLEFIN

(57) Provided are a catalyst for α-olefin production and a method of using the catalyst for producing α-olefins, of which the advantages are that the catalyst exhibits high activity of ethylene oligomerization and the amount of the side products such as heavy components and waxy components that may be produced in the method is reduced. In one aspect, the catalyst is prepared by contacting (a) any of clay, clay minerals and ion-exchange layer compounds with (b) a transition metal complex of which the center metal is a transition metal of Groups 8 to 10 of the Periodic Table, for a period of at least 10 minutes, and the catalyst is used for ethylene oligomerization to produce α-olefins. In another aspect, the catalyst is prepared by contacting (a') any of clay, clay minerals or ion-exchange layer compounds having been processed with an alkylating, with (b) a transition metal complex of which the center metal is a transition metal of Groups 8 to 10 of the Periodic Table, wherein the component (a') is such that its suspension formed by adding an inert medium thereto releases at most 1 mmols, per gram of the component (a'), of the alkylating agent in the medium, and the catalyst is used for ethylene oligomerization to produce α-olefins.

EP 1 106 249 A1

## Description

### TECHNICAL FIELD

The present invention relates to a catalyst for the production of α-olefins and to a method for the production of α-olefins. More precisely, it relates to a catalyst for α-olefin production with which α-olefins can be efficiently produced at low costs, and also to a method for producing α-olefins through oligomerization of ethylene with the catalyst.

### BACKGROUND ART

For producing α-olefins through polymerization of ethylene, known is a process of using a nickel complex (Shell Higher Olefin Process: SHOP), and α-olefins are produced according to the process.

Recently, a method has been proposed for producing α-olefins through oligomerization of ethylene, for which is used a transition metal complex serving as a main catalyst along with an oxygen-containing organoaluminium compound such as aluminoxane or the like, or a boron compound such as perfluorotetraphenyl borate or the like that serves as a catalyst promoter. Concretely, for example, proposed is a method of using a metallocene catalyst that comprises a metallocene complex with a center metal of Zr combined with an aluminoxane, for producing α-olefins (EP 366,212).

Most recently, it has been found that ethylene is polymerized in the presence of an iron chelate complex (Chem. Commun., 1998, 849-850). This reference discloses a method of polymerizing ethylene, in which an iron chelate complex having a ligand bonded to the center metal iron via a nitrogen atom is used as a main catalyst and methylaluminoxane is used as a catalyst promoter. They say that the catalyst ensures high ethylene polymerization activity. Also disclosed is a method for producing α-olefins through oligomerization of ethylene.

However, the boron compound is problematic in that its production is difficult. On the other hand, in the method of using an oxygen-containing compound such as aluminoxane, the amount of the compound to be used must be at least hundreds times moles of that of the main catalyst to be used along with the compound. Therefore, the method is problematic in that the catalytic activity per the oxygen-containing compound used therein is low and the efficiency for producing α-olefins therein is low. In addition, still another problem with the method is that the reaction mixture contains many side products such as heavy components and waxy components.

The present invention has been made in consideration of the matters mentioned above, and its object is to provide a catalyst for α-olefin production and a method of using the catalyst for ethylene oligomerization to produce α-olefins, of which the advantages are that the catalyst exhibits high activity of ethylene oligomerization and the amount of the side products such as heavy components and waxy components that may be produced in the method is reduced.

### DISCLOSURE OF THE INVENTION

We, the present inventors have found that, when any of clay, clay minerals or ion-exchange layer compounds is used as a catalyst promoter and when a catalyst prepared by contacting the catalyst promoter with a transition metal complex, of which the center metal is a transition metal of Groups 8 to 10 of the Periodic Table, under a specific condition is used, then the above-mentioned object can be effectively attained. On the basis of this finding, we have completed the present invention.

We have further found that, when any of specifically-processed clay, clay minerals or ion-exchange layer compounds is used as a catalyst promoter and when a catalyst prepared by contacting the catalyst promoter with a transition metal complex, of which the center metal is a transition metal of Groups 8 to 10 of the Periodic Table, is used, then the above-mentioned object can be effectively attained. On the basis of this finding, we have completed the invention.

Specifically, the invention is summarized as follows:

1. A catalyst for α-olefin production, which is prepared by contacting (a) any of clay, clay minerals and ion-exchange layer compounds with (b) a transition metal complex of which the center metal is a transition metal of Groups 8 to 10 of the Periodic Table, for a period of at least 10 minutes.
2. The catalyst for α-olefin production of above 1, wherein the component (a) has a volume-average particle size of at most 10 μm.
3. The catalyst for α-olefin production of above 2, wherein the particles of the component (a) having a volume-average particle size of at most 3.0 μm account for at least 10 % by weight of the total of the component (a).
4. The catalyst for α-olefin production of any of above 1 to 3, wherein the component (b) is a transition metal complex of a general formula (1):

$$L^1L^2L^3MX^1{}_mY^1{}_n \quad (1)$$

where M represents a transition metal of Groups 8 to 10 of the Periodic Table; at least one of $L^1$ to $L^3$ represents a ligand capable of bonding to the transition metal via a hetero atom, and these may be bonded to each other to form a ring; $X^1$ and $Y^1$ each represent a covalent-bonding or ionic-bonding ligand, and they may be the same or different; m and n each indicate 0 or a positive integer, and the sum of m and n is 0, 1, 2 or 3, depending on the valency of M.

5. The catalyst for α-olefin production of above 4, wherein all of $L^1$ to $L^3$ in formula (1) are ligands capable of bonding to the transition metal via a hetero atom.

6. The catalyst for α-olefin production of any of above 1 to 5, wherein the component (b) is a transition metal complex having a polydentate ligand that bonds to the transition metal via a hetero atom.

7. The catalyst for α-olefin production of any of above 1 to 6, wherein the component (a) is an organosilane compound-processed clay, clay mineral or ion-exchange layer compound.

8. The catalyst for α-olefin production of above 7, wherein the organosilane compound is represented by a general formula:

$$R_nSiX_{4-n}$$

where R represents a substituent in which the atom directly bonding to the silicon atom is a carbon, silicon or hydrogen atom; X represents a substituent in which the atom directly bonding to the silicon atom is a halogen, oxygen or nitrogen atom; a plurality of R's and X's, if any, may be the same or different; n indicates an integer of from 1 to 3.

9. A method for producing α-olefins, which comprises oligomerizing ethylene in the presence of the catalyst for α-olefin production of any of above 1 to 8.

10. A catalyst for α-olefin production, which is prepared by contacting (a') any of clay, clay minerals or ion-exchange layer compounds having been processed with an alkylating agent, with (b) a transition metal complex of which the center metal is a transition metal of Groups 8 to 10 of the Periodic Table, wherein the component (a') is such that its suspension formed by adding an inert medium thereto releases at most 1 mmols, per gram of the component (a'), of the alkylating agent in the medium.

11. The catalyst for α-olefin production of above 10, wherein the component (a') has a volume-average particle size of at most 10 μm.

12. The catalyst for α-olefin production of above 11, wherein the particles of the component (a') having a volume-average particle size of at most 3.0 μm account for at least 10 % by weight of the total of the component (a').

13. The catalyst for α-olefin production of any of above 10 to 12, wherein the component (b) is a transition metal complex of above formula (1).

14. The catalyst for α-olefin production of above 13, wherein all of $L^1$ to $L^3$ in formula (1) are ligands capable of bonding to the transition metal via a hetero atom.

15. The catalyst for α-olefin production of any of above 10 to 14, wherein the component (b) is a transition metal complex having a polydentate ligand that bonds to the transition metal via a hetero atom.

16. The catalyst for α-olefin production of any of above 10 to 15, wherein the component (a') is any of clay, clay minerals or ion-exchange layer compounds having been processed with an organosilane compound and then with an alkylating agent.

17. The catalyst for α-olefin production of above 16, wherein the organosilane compound is represented by a general formula:

$$R_nSiX_{4-n}$$

where R, X and n have the same meanings as above.

18. A method for producing α-olefins, which comprises oligomerizing ethylene in the presence of the catalyst for α-olefin production of any of above 10 to 17.

## BEST MODES OF CARRYING OUT THE INVENTION

The invention is described in detail hereinunder.

The invention includes a first aspect and a second aspect. The first aspect of the invention includes a catalyst

for α-olefin production, in which is used any of clay, clay minerals or ion-exchange layer compounds as a catalyst promoter and which is prepared by contacting the catalyst promoter with a transition metal complex of which the center metal is a transition metal of Groups 8 to 10 of the Periodic Table, under a specific condition; and a method for producing α-olefins through oligomerization of ethylene with the catalyst.

The second aspect includes a catalyst for α-olefin production, in which is used any of specifically-processed clay, clay minerals or ion-exchange layer compounds as a catalyst promoter and which is prepared by contacting the catalyst promoter with a transition metal complex of which the center metal is a transition metal of Groups 8 to 10 of the Periodic Table; and a method for producing α-olefins through oligomerization of ethylene with the catalyst.

α-olefins referred to herein are meant to indicate polymers having a molecular weight of at most 10,000, and they differ in physical properties and applications from ordinary high-molecular substances having a higher molecular weight and therefore having characteristics intrinsic to them. Accordingly, the catalyst for producing such α-olefins shall differ in the catalytic function from that for producing ordinary high-molecular substances. Consequently, it is not true that ordinary catalysts for producing high-molecular substances could be directly used for producing α-olefins.

The first aspect of the invention is described.

The catalyst for α-olefin production of the first aspect is prepared by contacting (a) any of clay, clay minerals and ion-exchange layer compounds (hereinafter referred to as clay and others, when appropriate) with (b) a transition metal complex of which the center metal is a transition metal of Groups 8 to 10 of the Periodic Table, for a period of at least 10 minutes. The components (a) and (b) are described concretely.

<u>Component (a)</u>

The component (a) is any of clay, clay minerals or ion-exchange layer compounds. Clay is an aggregate of fine hydrous silicate minerals. It is plastic when kneaded with a suitable amount of water, and is rigid when dried. When baked at high temperatures, it is sintered. Clay minerals are hydrous silicates which are the essential components constituting clay. The component (a) may be any of such clay and clay minerals, which may be natural ones or synthetic products.

Ion-exchange layer compounds for (a) are characterized by the crystal structure of such that a plurality of crystal planes are laminated in parallel with each other via weak bonding therebetween, in which the ions are exchangeable. Some clay minerals are ion-exchange layer compounds.

Specific examples of the component (a) are mentioned. For example, phyllosilicic acid compounds belong to clay minerals. Phyllosilicic acid compounds include phyllosilicic acid and phyllosilicates. As natural phyllosilicates, known are montmorillonite, saponite and hectorite of the smectite family; illite and sericite of the mica family; and mixed layer minerals of smectites and micas, or those of micas and vermiculites. As synthetic products, known are fluorotetrasilicon mica, laponite, smectone, etc. Also mentioned are ion-exchanging layer compounds having a layered crystal structure, such as α-$Zr(HPO_4)_2$, γ-$Zr(HPO_4)_2$, α-$Ti(HPO_4)_2$, γ-$Ti(HPO_4)_2$, etc. These are not clay minerals.

Examples of clay and clay minerals which do not belong to ion-exchange layer compounds include clay having a low montmorillonite content and referred to as bentonite; kibushi clay containing montmorillonite and many other components; gairome clay; sepiolite and palygorskite having a fibrous morphology; and amorphous or low-crystalline allophane, imogolite, etc.

Among the component (a), clay minerals are preferable and concretely phyllosilicic acid compounds are preferable. Furthermore phyllsoilicates belong to smectite family are preferable, and montmorillonite is especially preferable.

The component (a) may comprise one or more of the substances mentioned above either singly or as combined.

Preferably, the component (a) has a volume-average particle size of at most 10 μm, more preferably at most 3.0 μm. As a rule, particles have a particle size distribution. For its particle size distribution, preferably, the component (a) has a volume-average particle size of at most 10 μm and the particles thereof having a volume-average particle size of at most 3.0 μm account for at least 10 % by weight of the component (a). More preferably, the component (a) has a volume-average particle size of at most 10 μm and the particles thereof having a volume-average particle size of at most 1.5 μm account for at least 10 % by weight of the component (a). For measuring the volume-average particle size and the particle size distribution of the component (a), for example, used is an apparatus for measuring the particle size of particles from the laser beam transmittance through particles (for example, GALAI Production's CIS-1). Briefly, for example, one gram of a sample of the component (a) is suspended in one liter of water, and stirred at room temperature for 20 hours to give a slurry, and the slurry is tested in the apparatus for measuring the particle size of the sample. For obtaining particles of the component (a) having a volume-average particle size of at most 10 μm, employable is any known grinding method.

The component (a) may be used herein directly as it is, or after water is adsorbed thereto, or after it is dewatered under heat. However, it is desirable that clay, clay minerals and ion-exchange layer compounds for the component

(a) are chemically treated for the purpose of removing impurities from them or for modifying their structures and functions to those more preferred for catalytic ingredients, prior to being contacted with the component (b).

The chemical treatment includes surface treatment to remove impurities from the surface of clay and others and treatment to modify the crystal structure of clay and others. Concretely, it includes acid treatment, alkali treatment, salt treatment, organic treatment, etc.

Through acid treatment, impurities can be removed from the surface of clay and others. Through it, cations such as aluminium, iron, magnesium and the like are released from the crystal structure of clay and others to thereby enlarge the surface area thereof. Through alkali treatment, the crystal structure of clay and others can be modified to any other desired one.

Through salt treatment or organic treatment, ionic complexes, molecular complexes, organic complexes and the like may be formed on the surface of clay and others or inside them, whereby the surface area and the layer-to-layer space of the thus-treated clay and others can be changed to desired ones. For example, owing to their ion-exchanging ability, the interlayer exchangeable ions in the compounds maybe exchanged with any other bulky ions to give layer substances having enlarged interlayer spaces.

Clay and others thus chemically treated in the manner mentioned above may be optionally processed with an organosilane compound for further increasing their catalytic activity. The organosilane compound to be used for this treatment includes those of a general formula:

$$R_nSiX_{4-n}$$

wherein R represents a substituent in which the atom directly bonding to the silicon atom is a carbon, silicon or hydrogen atom; X represents a substituent in which the atom directly bonding to the silicon atom is a halogen, oxygen or nitrogen atom; a plurality of R's and/or X's, if any, may be the same or different; n indicates an integer of from 1 to 3.

The organosilane compound further includes bis-silyl compounds of a general formula:

$$X_{4-n}Si(CH_2)_mSiX_{4-n}$$

wherein m indicates from 1 to 10; and n indicates from 1 to 3, and also polycyclic polysiloxanes, polysilazanes, etc.

Specific examples of the organosilane compounds of above formulae are trialkylsilyl chlorides such as trimethylsilyl chloride, triethylsilyl chloride, triisopropylsilyl chloride, t-butyldimethylsilyl chloride, t-butyldiphenylsilyl chloride, phenethyldimethylsilyl chloride, etc.; dialkylsilyl dichlorides, diarylsilyl dichlorides and alkylarylsilyl dichlorides such as dimethylsilyl dichloride, diethylsilyl dichloride, diisopropylsilyl dichloride, di-n-hexylsilyl dichloride, dicyclohexylsilyl dichloride, docosylmethylsilyl dichloride, bis (phenethyl) silyl dichloride, methylphenethylsilyl dichloride, diphenylsilyl dichloride, dimesitylsilyl dichloride, ditolylsilyl dichloride, etc.

Also usable herein are other silyl halides to be derived from the above-mentioned compounds by substituting the chloride moiety therein with any other halogens; disilazanes such as bis(trimethylsilyl)amide, bis(triethylsilyl)amide, bis(triisopropylsilyl)amide, bis(dimethylethylsilyl)amide, bis(diethylmethylsilyl)amide, bis(dimethylphenylsilyl)amide, bis(dimethyltolylsilyl)amide, bis(dimethylmenthylsilyl)amide, etc.; trialkylsilyl hydroxides such as trimethylsilyl hydroxide, triethylsilyl hydroxide, triisopropylsilyl hydroxide, tertbutyldimethylsilyl hydroxide, phenethyldimethylsilyl hydroxide, etc.; polysilanols generally known as peralkylpolysiloxypolyols; bissilyls such as bis(methyldichlorosilyl)methane, 1,2-bis(methyldichlorosilyl)ethane, bis(methyldichlorosilyl)octane, bis(triethoxysilyl)ethane, etc.; silane hydrides such as dimethylchlorosilane, (N,N-dimethylamino)dimethylsilane, diisobutylchlorosilane, etc. One or more of these organosilane compounds may be used herein either singly or as combined.

Of the organosilane compounds, preferred are those having at least one alkyl group directly bonding to the silicon atom. Favorably used are alkylsilyl halides, more favorably dialkylsilyl dihalides. Treatment with such an organosilane compound is preferably effected in the presence of water to ensure better results. In that case, water breaks the crystal structure (especially the layered crystal structure) of clay and others, thereby enhancing the contact efficiency between the organosilane compound and the thus-broken clay and others. Specifically, water expands the layer-to-layer spaces in the crystal structure of clay and others and therefore expands the secondary particles thereof having the thus-expanded, layered crystal structure, thereby promoting the diffusion of an organosilane compound into the surface of the thus-expanded particles.

The treatment is concretely described. First, water is added to the component (a) to prepare an aqueous colloidal solution of the component (a). Next, an organosilane compound such as that mentioned above is added to the thus-prepared, aqueous colloidal solution of the component (a), and stirred under heat, whereby the component (a) is processed with the organosilane compound. This treatment may be effected at room temperature to 200°C. Preferably, it is effected at a temperature around 100°C at which it will be facilitated. To the contrary, treatment at

around room temperature will takes a longer treatment time, but treatment at a temperature higher than 100°C will require a pressure device. The treatment time will vary, depending on the type of the component (a) to be treated and on the treatment temperature, but may fall between 0.5 and 24 hours.

The amount of the organosilane compound to be used for treating the component (a) may fall between 0.001 and 1000 mols, but preferably between 0.01 and 100 mols, in terms of the silicon atom in the organosilane compound, per kg of the component (a).

Component (b)

The component (b) is described. The component (b) serves as a main catalyst, and it is a complex with a transition metal of Groups 8 to 10 of the Periodic Table. The complex may be selected from a variety of transition metal complexes covering a broad range.

For the component (b), a complex with a transition metal of Groups 8 to 10 of the Periodic Table, preferred are transition metal complexes of a general formula (1):

$$L^1L^2L^3MX^1{}_mY^1{}_n \qquad (1)$$

In formula (1), M represents a transition metal of Groups 8 to 10 of the Periodic Table, concretely including iron, cobalt, nickel, palladium, platinum, etc. Preferred are iron and cobalt.

At least one of $L^1$ to $L^3$ represents a ligand capable of bonding to the transition metal M via a hetero atom, and these may be bonded to each other to form a ring. The hetero atom includes nitrogen, oxygen, sulfur and other atoms except carbon atom. Of these, preferred is a nitrogen atom. Preferably, the nitrogen atom forms an unsaturated bond with a carbon atom. More preferably, $L^1$ to $L^3$ are ligands having C=N structure units. It is also desirable that all of $L^1$ to $L^3$ are ligands capable of bonding to the transition metal M via a hetero atom.

$X^1$ and $Y^1$ each represent a covalent-bonding or ionic-bonding ligand, concretely including a hydrogen atom, a halogen atom, a hydrocarbon group having from 1 to 20 (preferably from 1 to 10) carbon atoms, an alkoxy group having from 1 to 20 (preferably from 1 to 10) carbon atoms, an amino group, a phosphorus-containing hydrocarbon group having from 1 to 20 (preferably from 1 to 12) carbon atoms (e.g., diphenylphosphine group), a silicon-containing hydrocarbon group having from 1 to 20 (preferably from 1 to 12) carbon atoms, and a halogen-containing boron anion (e.g., $BF_4^-$). Of those, preferred are a halogen atom, and a silicon-containing hydrocarbon group having from 1 to 12 carbon atoms. $X^1$ and $Y^1$ may be the same or different.

m and n each indicate 0 or a positive integer, and the sum of m and n is 0, 1, 2 or 3, depending on the valency of M.

More preferably, the transition metal complex of formula (1) has a polydentate ligand bonding to the transition metal M via a hetero atom. The polydentate is preferably a tridentate. Especially preferred are transition metal complexes having a nitrogen-containing tridentate ligand. For these, for example, mentioned are complexes of a general formula (2):

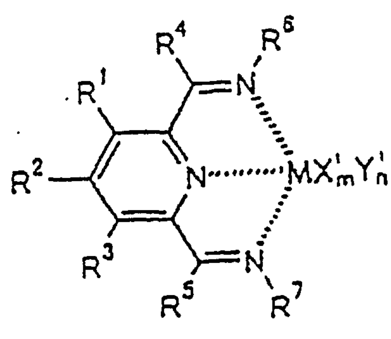

(2)

wherein $R^1$ to $R^5$ each independently represent a hydrogen atom, or a hydrocarbon group having from 1 to 20 carbon atoms, and they may be bonded to each other to form a ring; $R^6$ and $R^7$ each independently represent an aliphatic hydrocarbon group having from 1 to 20 carbon atoms, or an aromatic group having from 7 to 20 carbon atoms in total and having a hydrocarbon group bonded to its ring; $X^1$ and $Y^1$ each represent a covalent-bonding or ionic-bonding

ligand, and they may be the same or different; M represents a transition metal of Groups 8 to 10 of the Periodic Table; m and n each indicate 0 or a positive integer, and the sum of m and n is 0, 1, 2 or 3, depending on the valency of M.

In formula (2), the hydrocarbon group having from 1 to 20 carbon atoms for $R^1$ to $R^5$ includes, for example, a linear or branched alkyl group having from 1 to 20 carbon atoms, a cycloalkyl group having from 3 to 20 carbon atoms, an aryl group having from 6 to 20 carbon atoms, an aralkyl group having from 7 to 20 carbon atoms, etc. The linear or branched alkyl group having from 1 to 20 carbon atoms includes, for example, a methyl group, an ethyl group, an n-propyl group, an isopropyl group, an n-butyl group, an isobutyl group, a sec-butyl group, a tert-butyl group, all types of pentyl groups, all types of hexyl groups, all types of octyl groups, all types of decyl groups, all types of tetradecyl groups, all types of hexadecyl groups, all types of octadecyl groups, etc. The cycloalkyl group having from 3 to 20 carbon atoms includes, for example, a cyclopentyl group, a cyclohexyl group, a cyclooctyl group, etc. The ring of the cycloalkyl group may have suitable substituents such as a lower alkyl group, etc. The aryl group having from 6 to 20 carbon atoms includes, for example, a phenyl group, a tolyl group, a xylyl group, a naphthyl group, a methylnaphthyl group, etc. The aralkyl group having from 7 to 20 carbon atoms includes, for example, a benzyl group, a phenethyl group, etc.

The aliphatic hydrocarbon group having from 1 to 20 for $R^6$ and $R^7$ in formula (2) includes, for example, those concretely mentioned for the linear or branched alkyl group having from 1 to 20 carbon atoms and the cycloalkyl group having from 3 to 20 carbon atoms for $R^1$ to $R^5$. For $R^6$ and $R^7$, the aromatic hydrocarbon group having from 7 to 20 carbon atoms in total and having a hydrocarbon group bonded to its ring includes, for example, those having at least one linear, branched or cyclic alkyl group with from 1 to 10 carbon atoms bonded to the aromatic ring of, for example, a phenyl group, a naphthyl group, etc. For these, preferred is an aromatic group having a hydrocarbon group bonded to its ring; and more preferred is a 2,4-dimethylphenyl group.

To $X^1$ and $Y^1$ in formula (2), the same as in formula (1) mentioned above shall apply. The halogen atom for $X^1$ and $Y^1$ includes chloride, bromine and iodine atoms. Of these, preferred is a chlorine atom. The hydrocarbon group having from 1 to 20 carbon atoms includes, for example, those mentioned hereinabove for $R^1$ to $R^5$. M, m and n in formula (2) are the same as in formula (1).

Concretely, the component (b) includes iron or cobalt complexes having a ligand selected from 2,6-diacetylpyridine-bisimine compounds, 2,6-diamidopyridine compounds, 2,6-diacetylaniline-bisimine compounds, etc. Above all, especially preferred are iron complexes having a ligand of a 2,6-diacetylpyridine-bisimine compound. The complexes include metal complexes of a general formula (3):

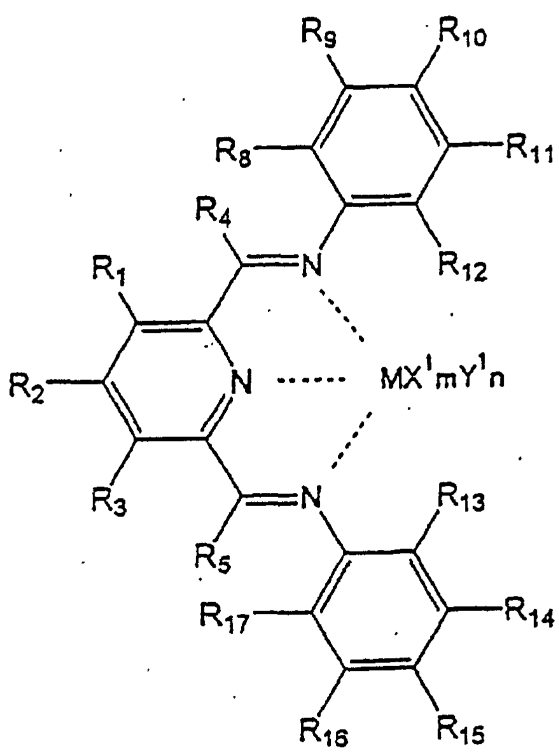

(3)

wherein M represents a transition metal of Groups 8 to 10; $R^1$ to $R^5$, $R^9$ to $R^{11}$ and $R^{14}$ to $R^{16}$ each independently

represent a hydrogen atom, a hydrocarbon group, a substituted hydrocarbon group, or an inert functional group; $R^8$, $R^{12}$, $R^{13}$ and $R^{17}$ each represent a hydrogen atom, a hydrocarbon group, a substituted hydrocarbon group, or a inert functional group; any two adjacent groups of $R^8$ to $R^{17}$ may be bonded to each other to form a ring; $X^1$ and $Y^1$ each represent a covalent-bonding or ionic-bonding ligand, and they may be the same or different; m and n each indicate 0 or a positive integer, and the sum of m and n is 0, 1, 2 or 3, depending on the valency of M.

In the formula, M is a transition metal of Groups 8 to 10, preferably iron or cobalt.

$R^1$ to $R^5$, R9 to $R^{11}$, and $R^{14}$ to $R^{16}$ each independently represent a hydrogen atom, a hydrocarbon group, a substituted hydrocarbon group, or an inert functional group; and $R^8$, $R^{12}$, $R^{13}$ and $R^{17}$ each represent a hydrogen atom, a hydrocarbon group, a substituted hydrocarbon group, or a inert functional group. The hydrocarbon group may have from 1 to 30 carbon atoms, including, for example, linear hydrocarbon groups having from 1 to 30 carbon atoms, such as a methyl group, an ethyl group, an n-propyl group, etc.; branched hydrocarbon groups having from 3 to 30 carbon atoms, such as an isopropyl group, a sec-butyl group, a tert-butyl group, etc.; cycloaliphatic hydrocarbon groups having from 3 to 30 carbon atoms, such as a cyclopentyl group, a cyclohexyl group, etc.; aromatic hydrocarbon groups having from 6 to 30 carbon atoms, such as a phenyl group, a naphthyl group, etc.

The substituted hydrocarbon group may be derived from the hydrocarbon group mentioned above, by substituting at least one hydrogen atom therein with a substituent. For example, it may be such a substituted hydrocarbon group having from 1 to 30 carbon atoms. The substituent includes a hydrocarbon group, a halogen atom, a hetero atom-containing hydrocarbon group, etc. The hydrocarbon group for the substituent may be any of the hydrocarbon groups mentioned above. The halogen atom includes chlorine, bromine, fluorine and iodine groups. The hetero atom includes nitrogen, oxygen, sulfur and other atoms. The substituted hydrocarbon group may contain a heteroaromatic ring.

The inert functional group may be any inert functional group except the hydrocarbon group and the substituted hydrocarbon group mentioned above. Concretely, it includes a halogen atom (fluorine, chlorine, bromine and iodine atoms), and an ether of -OR (in which R indicates a hydrocarbon group or a substituted hydrocarbon group). In the compounds of the formula where such groups of $R^4$, $R^5$, $R^8$, $R^{12}$, $R^{13}$ and $R^{17}$ are adjacent to M, it is difficult to say that the groups are coordinated with M. In these compounds, therefore, the groups are referred to as inert functional groups.

$R^8$ may also be a primary carbon-having group, a secondary carbon-having group, or a tertiary carbon-having group. In case where $R^8$ is a primary carbon-having group, at most two (including zero) of $R^{12}$, $R^{13}$ and $R^{17}$ may be primary carbon-having groups, and the remaining others may be hydrogen atoms. In case where $R^8$ is a secondary carbon-having group, at most one (including zero) of $R^{12}$, $R^{13}$ and $R^{17}$ may be a primary carbon-having group, and the remaining others may be hydrogen atoms. In case where $R^8$ is a tertiary carbon-having group, $R^{12}$, $R^{13}$ and $R^{17}$ may be hydrogen atoms.

Preferred embodiments of the cases as above are described below.

$R^8$ is any of a primary carbon-having group, a secondary carbon-having group, or a tertiary carbon-having group; and when $R^8$ is a primary carbon-having group, at most two (including zero) of $R^{12}$, $R^{13}$ and $R^{17}$ are primary carbon-having groups, and the remaining others are hydrogen atoms. When $R^8$ is a secondary carbon-having group, at most one (including zero) of $R^{12}$, $R^{13}$ and $R^{17}$ is a primary carbon-having group, the remaining others are hydrogen atoms. When $R^8$ is a tertiary carbon-having group, $R^{12}$, $R^{13}$ and $R^{17}$ are hydrogen atoms. Any two adjacent groups of $R^8$ to $R^{17}$ may be bonded to each other to form a ring.

The primary carbon referred to herein is represented by a formula, $-CH_2$---. In the formula, any atom may be bonded to ---. For example, the atom bonding to it includes a hydrogen atom, a halogen atom, a carbon atom, an oxygen atom, a sulfur atom, etc. To the atom, any of hydrogen atoms, hydrocarbon groups, substituted hydrocarbon groups and inert functional groups may be bonded. For the hydrocarbon groups, substituted hydrocarbon groups and inert functional groups, examples of the groups mentioned hereinabove are referred to. The group of $-CH_2$--- includes, for example, $-CH_3$, $-CH_2CH(CH_3)_2$, $-CH_2Cl$, $-CH_2C_6H_5$, $-OCH_3$, $-CH_2OCH_3$, etc.

The secondary carbon referred to herein is represented by a formula:

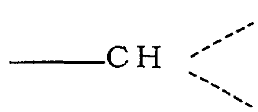

In this, atoms except hydrogen or groups are bonded to ---. The atoms and the groups may be the same or different. The groups include hydrocarbon groups, substituted hydrocarbon groups, and inert functional groups such as those mentioned hereinabove. The secondary carbon-having group of the formula includes, for example, $-CH(CH_3)_2$, $-CHCl_2$, $-CH(C_6H_5)_2$, a cyclohexyl group, $-CH(CH_3)OCH_3$, $-CH=CHCH_3$, etc.

The tertiary carbon referred to herein is represented by a formula:

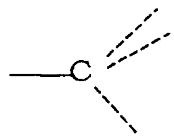

In this, atoms except hydrogen or groups are bonded to ---. The atoms and the groups may be the same or different. The groups include hydrocarbon groups, substituted hydrocarbon groups, and inert functional groups such as those mentioned hereinabove. The tertiary carbon-having group of the formula includes, for example, $-C(CH_3)_3$, $-CCl_3$, $-C(C_6H_5)_3$, $-C(CH_3)_2OCH_3$, $-C\equiv CH$, $-C(CH_3)_2CH=CH_2$, a 1-adamantyl group, etc.

$X^1$ and $Y^1$ in formula (3) have the same meanings as in formula (1). The halogen atom for $X^1$ and $Y^1$ includes chlorine, bromine and iodine atoms. Above all, preferred is a chlorine atom. m and n have the same meanings as in formula (1).

Preferred combinations of $R^1$ to $R^5$, $R^8$ to $R^{17}$, and $X^1$ and $Y^1$ in formula (3) are mentioned below.

$R^4$ and $R^5$ each are a methyl group or a hydrogen atom; $R^1$, $R^2$ and $R^3$ are all hydrogen atoms; $R^9$, $R^{10}$, $R^{11}$, $R^{14}$, $R^{15}$ and $R^{16}$ are all hydrogen atoms; and $R^{12}$ and $R^{17}$ are independently a methyl group, an ethyl group, a propyl group or an isopropyl group, and, more preferably, $R^{12}$ and $R^{17}$ are both methyl or ethyl groups. $X^1$ and $Y^1$ each are a monovalent anion, more preferably selected from hydrogen and a silicon-containing hydrocarbon group having from 1 to 12 carbon atoms.

Also preferred are the following combinations. $R^8$ is a primary carbon-having group, $R^{12}$ is a primary carbon-having group, and $R^{13}$ and $R^{17}$ are hydrogen atoms. $R^8$ is a secondary carbon-having group, $R^{12}$ is a primary carbon-having group or a secondary carbon-having group, but more preferably a secondary carbon-having group, and $R^{13}$ and $R^{17}$ are hydrogen atoms. $R^8$ is a tertiary carbon-having group, and $R^{12}$, $R^{13}$ and $R^{17}$ are hydrogen atoms.

More preferred combinations of $R^4$, $R^5$ and $R^8$ to $R^{17}$ in formula (3) are mentioned below.

$R^4$ and $R^5$ are methyl groups; $R^8$, $R^9$, $R^{10}$, $R^{11}$, $R^{13}$, $R^{14}$, $R^{15}$ and $R^{16}$ are all hydrogen atoms; and $R^{12}$ and $R^{17}$ are both methyl groups.

$R^4$ and $R^5$ are methyl groups; $R^8$, $R^9$, $R^{11}$, $R^{13}$, $R^{14}$, and $R^{16}$ are all hydrogen atoms; and $R^{10}$, $R^{12}$, $R^{15}$ and $R^{17}$ are all methyl groups.

$R^4$ and $R^5$ are methyl groups; $R^8$, $R^9$, $R^{10}$, $R^{11}$, $R^{13}$, $R^{14}$, $R^{15}$ and $R^{16}$ are all hydrogen atoms; and $R^{12}$ and $R^{17}$ are both ethyl groups.

$R^4$ and $R^5$ are methyl groups; $R^8$, $R^9$, $R^{10}$, $R^{11}$, $R^{13}$, $R^{14}$, $R^{15}$ and $R^{16}$ are all hydrogen atoms; and $R^{12}$ and $R^{17}$ are both isopropyl groups.

$R^4$ and $R^5$ are methyl groups; $R^8$, $R^9$, $R^{10}$, $R^{11}$, $R^{13}$, $R^{14}$, $R^{15}$ and $R^{16}$ are all hydrogen atoms; and $R^{12}$ and $R^{17}$ are both n-propyl groups.

$R^4$ and $R^5$ are methyl groups; $R^8$, $R^9$, $R^{10}$, $R^{11}$, $R^{13}$, $R^{14}$, $R^{15}$ and $R^{16}$ are all hydrogen atoms; and $R^{12}$ and $R^{17}$ are both chlorine atoms.

$R^4$ and $R^5$ are methyl groups; $R^8$, $R^9$, $R^{10}$, $R^{11}$, $R^{13}$, $R^{14}$, $R^{15}$ and $R^{16}$ are all hydrogen atoms; and $R^{12}$ and $R^{17}$ are both trifluoromethyl groups.

In these cases, $X^1$ and $Y^1$ are preferably selected from chlorine, bromine and a silicon-containing hydrocarbon group having from 1 to 12 carbon atoms, and are more preferably chlorine atoms.

One embodiment of producing the transition metal compounds of formula (3) is mentioned. A ketone compound of a general formula (4):

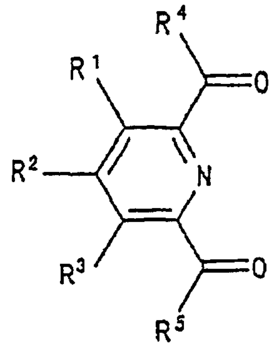

(4)

is reacted with $H_2NR^{18}$ or $H_2NR^{19}$. In the process, $R^{18}$ and $R^{19}$ each are a hydrocarbon group, preferably an aromatic hydrocarbon group. In the reaction, an organic acid such as formic acid or the like may be used, and it serves as a catalyst. The compound produced in the process is then reacted with a halide of a transition metal M (for example, a metal halide, etc.) to give the intended transition metal compound.

One or more of the compounds mentioned above may be used for the component (b) either singly or as combined.

Next described is a method for producing the catalyst for α-olefin production, which comprises contacting the components (a) and (b) with each other for a period of at least 10 minutes.

A catalyst prepared by separately adding the component (a) and the component (b) one after another to the polymerization system of ethylene could not readily express its catalytic function in the polymerization system. We, the present inventors have assiduously studied the problem of how to readily express the polymerization activity of the catalyst, and, as a result, have found that, when the component (a) and the component (b) are kept in contact with each other at a relatively low temperature for a prolonged period of time, then the polymerization activity of the resulting catalyst can be greatly increased. In addition, we have further found that the particle morphology of the clay, clay mineral or ion-exchange layer compound for the component (a) to be contacted with the component (b) has significant influences on the activation speed of the complex (this indicates the time which the catalyst takes for expressing its catalytic function) and on the ethylene polymerization activity of the catalyst formed from the components. A relatively low temperature referred to herein will fall between 0 and 80°C, but preferably between room temperature and 50°C. The time for which the components (a) and (b) are kept in contact with each other is at least 10 minutes, preferably at least 15 minutes, more preferably at least 1 hour, even more preferably at least 12 hours. The pressure under which the components (a) and (b) are kept in contact with each other may fall generally between ordinary pressure and about 10 MPa·G so or, but preferably between ordinary pressure and 4 MPa·G.

The treatment for contacting the two components with each other is preferably effected in an inert gas such as argon, nitrogen or the like. Also preferably, it is effected in a hydrocarbon solvent such as pentane, hexane, heptane, toluene, xylene or the like. Still preferably, the system for the treatment does not contain water or a compound having an active hydrogen of, for example, a hydroxyl group, an amino group or the like that is harmful to the catalyst. For this, it is desirable that the system for the treatment is pre-treated with an alkylating agent of the component (c), which will be described hereinunder, to thereby remove water and the active hydrogen-having compound from it. Concretely, the component (a) and the component (b) are contacted with each other in the presence of the component (c) to prepare the catalyst. However, when the component (c) is remained excessively in the system, it may exert a bad influence to the catalyst activity. The required amount of the component (c) used in the contact system of components (a) and (b) is sufficient to remove water and the active hydrogen-having compound from it. The component (c) is not always needed to be in the system where the catalyst is prepared.

Next, the component (c) is described. Component (c) is an alkylating agent. For the alkylating agent for the component (c), usable are oranozinc compounds and organomagnesium compounds, but preferred are inexpensive and easily available organoaluminium compounds. Concretely mentioned are trialkylaluminiums such as trimethylaluminium, triethylaluminium, tripropylaluminium, triisobutylaluminium, tri-tert-butylaluminium, etc.; halogen- or alkoxy group-having alkylaluminiums such as dimethylaluminium chloride, diethylaluminium chloride, dimethylaluminium methoxide, diethylaluminium ethoxide, etc.; aluminoxanes such as methylaluminoxane, ethylaluminoxane, isobutylaluminoxane, etc. Of those, preferred are trialkylaluminiums, and more preferred is triisobutylaluminium.

Component (c) is used to prepare the component (a'), which will be described hereinafter. The component (c) is optionally used to prepare a α-olefin production catalyst of the present invention. Further, the component (c) is preferably used for the α-olefin production method of the present invention.

Regarding the ratio of the component (a) to the component (b), the amount of the transition metal complex

of the component (b) may fall between 0.0001 and 10 mmols, but preferably between 0.001 and 0.1 mmol, relative to the unit weight (gram) of the component (a) of clay and others.

The treatment for contacting the components with each other to prepare the catalyst may be effected in a separate reactor for catalyst preparation, or may be in a polymerization reactor. The condition including temperature, pressure and time for the treatment of catalyst preparation in the presence of the component (c) may be the same as that mentioned above for the treatment of catalyst preparation in the absence of it. More preferably, however, the treatment is effected at a temperature not higher than the boiling point of the solvent used, under a gauge pressure of at most 4.0 MPa, and for a period of at least 10 minutes. Under the preferred condition, the catalytic function of the catalyst prepared will be better.

Last described is the method for producing α-olefins of the first aspect of the invention. In the method for producing α-olefins of the first aspect of the invention, ethylene is oligomerized in the presence of the catalyst having been prepared in the manner as above, optionally along with the component (c). The amount of the component (c) may fall generally between 0.1 and 1,000 mmols, but preferably between 1 and 100 mmols, relative to the unit weight (gram) of the component (a) of clay and others.

The mode of oligomerization is not specifically defined, including, for example, solution reaction to be effected in a solvent, liquid-phase non-solvent reaction to be effected substantially in the absence of a solvent, gas-phase reaction, etc. In the invention, any of those methods is employable. The reaction may be in any mode of continuous reaction or batch reaction. The solvent, if used, may be a hydrocarbon solvent such as pentane, hexane, heptane, cyclohexane, benzene, toluene, etc. One or more of these solvents may be used either singly or as combined. Regarding the amount of the catalyst to be used in the reaction in the presence of such a solvent, it is desirable that the amount of the component (b) falls generally between 0.1 and 100 μmols, but preferably between 1 and 20 μmols in one liter of the solvent, in view of the reaction activity.

The reaction condition is not specifically defined. The reaction temperature may fall generally between -78 and 200°C, but preferably between room temperature and 150°C. The ethylene pressure in the reaction system may fall generally between normal pressure and 15 MPa, but preferably between normal pressure and 5 MPa. The molecular weight of the product to be produced through the reaction may be controlled in any known manner, for example, by suitably changing the reaction temperature or pressure.

Next described in the second aspect of the invention. The second aspect includes a catalyst for α-olefin production, in which is used any of specifically-processed clay, clay minerals or ion-exchange layer compounds (these will be referred to as clay and others, like those for the component (a) mentioned above), as a catalyst promoter for the component (a') and which is prepared by contacting the catalyst promoter with a transition metal complex of which the center metal is a transition metal of Groups 8 to 10 of the Periodic Table for the component (b); and a method for producing α-olefins through oligomerization of ethylene with the catalyst.

First described is the component (a'). The component (a') is any of clay, clay minerals or ion-exchange layer compounds having been processed with an alkylating agent, wherein the component (a') is such that its suspension formed by adding an inert medium thereto releases at most 1 mmols, per gram of the component (a'), of the alkylating agent in the medium. The α-olefin production catalyst of the second aspect is prepared by contacting the component (a') with (b) a transition metal complex of which the center metal is a transition metal of Groups 8 to 10 of the Periodic Table. The other preparation method of component (a') is that clay and others is processed with an alkylating agent and then washed with an inert medium to remove the excessive alkylating agent.

Comparing the component (a) and the component (a') for use in the invention, the two differ from each other in that the latter indispensably requires the treatment with an alkylating agent and a released amount of the alkylating agent is limited. Therefore, the description of the above-mentioned Component (a) maybe applied to the component (a') . Concretely, the component (a') preferably has a volume-average particle size of at most 10 μm, and more preferably, it has a volume-average particle size of at most 3.0 μm account for at least 10 % by weight of the total of the component (a') . Still preferably, the component (a') is processed with an organosilane compound and then with an alkylating agent. For the organosilane compound for treating clay and others herein, preferably used are represented by a general formula "$R_nSiX_{4-n}$", where R, X and n have the same meanings as above.

For the alkylating agent for treating clay and others herein, preferably used are the compounds mentioned hereinabove for the component (c). Of those, preferred are organoaluminium compounds, more preferred are trialkylaluminiums, and especially preferred is triisobutylaluminium. In general, the alkylating agent is diluted with an inert solvent, and then contacted with clay and others. The inert solvent may be a hydrocarbon solvent such as pentane, hexane, heptane, cyclohexane, benzene, toluene, etc. The amount of the alkylating agent to be used for the treatment may fall generally between 0.01 and 1,000 mmols, but preferably between 0.1 and 100 mmols, relative to the unit weight (gram) of clay and others. Even when an excessive amount of the alkylating agent is used, it may be removed out of the system by washing clay and others having been treated with it. The temperature for the treatment preferably falls between 20 and 150°C, and more preferably falls between 70 and 110°C. During the treatment, it is desirable to agitate clay and others to thereby facilitate their contact with the alkylating agent.

In the second aspect of the invention, after clay and others are contacted with the alkylating agent, the excessive alkylating agent remaining in the inert medium used or in the processed clay and others must be removed in order to increase the catalytic activity of the catalyst. For this, in general, the processed clay and others are washed with an inert medium to remove the alkylating agent remaining therein. The washing medium to be used may be a hydrocarbon solvent such as pentane, hexane, heptane, cyclohexane, benzene, toluene, etc. The washing method is not specifically defined, and may be effected in any ordinary manner. For example, the slurry is agitated, and then kept static as it is, and thereafter the supernatant is removed. The ratio of the washing medium to clay and others to be washed with it, the agitation power, the time for agitation, the time for stationary treatment, and the washing temperature may be suitably determined. Preferably, the washing temperature falls between 20 and 150°C.

If the amount of the alkylating agent released in the inert medium is at most 1 mmols per gram of the processed clay and others, washing is not necessary. Washing the processed clay and others is continued until the amount of the alkylating agent released in the inert medium is reduced to be at most 1 mmols per gram of the washed clay and others. The amount of the alkylating agent released in the inert medium is preferably at most 0.1 mmols per gram of the processed or washed clay and others. Accordingly, washing the processed clay and others is continued until the amount of the alkylating agent released in the inert medium is reduced to be at most 0.1 mmols per gram of the washed clay and others.

The component (b) is the same as in the first aspect of the invention described hereinabove.

Next described is a method for producing the catalyst for α-olefin production, which comprises contacting the components (a') and (b).

In the catalyst production method, there is no limitation concerning the contact time of the components (a') and (b). However, the contact conditions, namely contact temperature, contact time, and contact pressure, mentioned in the catalyst production method using the components (a) and (b) are also preferable.

The treatment for contacting the components (a') and (b), with each other is preferably effected in an inert gas such as argon, nitrogen or the like. Also preferably, it is effected in a hydrocarbon solvent such as pentane, hexane, heptane, toluene, xylene or the like. Still preferably, the system for the treatment does not contain water or a compound having an active hydrogen of, for example, a hydroxyl group, an amino group or the like that is harmful to the catalyst. For this, it is desirable that the system for the treatment is pre-treated with an alkylating agent of the component (c) to thereby remove water and the active hydrogen-having compound from it. Concretely, the component (a') and the component (b) are contacted with each other in the presence of the component (c) to prepare the catalyst. However, when the component (c) is remained excessively in the system, it may exert a bad influence to the catalyst activity. It is enough to be present the component (c) to remove water and the active hydrogen-having compound from the system. The component (c) is not always needed to be in the system where the catalyst is prepared if it exists a very small amount of water and the active hydrogen-having compound.

For the component (c) using in the preparation of catalyst, preferably used are the compounds mentioned hereinabove for the component (c). Of those, preferred are organoaluminium compounds, more preferred are trialkylaluminiums, and especially preferred is triisobutylaluminium.

Regarding the ratio of the component (a') to the component (b), the amount of the transition metal complex of the component (b) may fall between 0.0001 and 10 mmols, but preferably between 0.001 and 0.1 mmol, relative to the unit weight (gram) of the component (a') of clay and others.

The treatment for contacting the components with each other to prepare the catalyst may be effected in a separate reactor for catalyst preparation, or may be in a polymerization reactor. The condition including temperature, pressure and time for the treatment of catalyst preparation in this aspect may be the same as that mentioned above for the treatment of catalyst preparation in the presence of the component (c) in the section of the first aspect. More preferably, however, the treatment is effected at a temperature not higher than the boiling point of the solvent used and under a gauge pressure of at most 4.0 MPa·G. Under the preferred condition, the catalytic function of the catalyst prepared will be better.

Last described is the method for producing α-olefins of the second aspect of the invention. In the method for producing α-olefins of the second aspect of the invention, ethylene is oligomerized in the presence of the catalyst having been prepared in the manner as above, optionally along with the component (c). The amount of the component (c) may fall generally between 0.1 and 1,000 mmols, but preferably between 1 and 100 mmols, relative to the unit weight (gram) of the component (a') of clay and others.

The mode of oligomerization is not specifically defined, including, for example, solution reaction to be effected in a solvent, liquid-phase non-solvent reaction to be effected substantially in the absence of a solvent, gas-phase reaction, etc. In the invention, any of those methods is employable. The reaction may be in any mode of continuous reaction or batch reaction. The solvent, if used, may be a hydrocarbon solvent such as pentane, hexane, heptane, cyclohexane, benzene, toluene, etc. One or more of these solvents may be used either singly or as combined. Regarding the amount of the catalyst to be used in the reaction in the presence of such a solvent, it is desirable that the amount of the component (b) falls generally between 0.1 and 100 μmols, but preferably between 1 and 20 μmols in

one liter of the solvent, in view of the reaction activity.

The reaction condition is not specifically defined. The reaction temperature may fall generally between -78 and 200°C, but preferably between room temperature and 150°C. The ethylene pressure in the reaction system may fall generally between normal pressure and 15 MPa, but preferably between normal pressure and 5 MPa. The molecular weight of the product to be produced through the reaction may be controlled in any known manner, for example, by suitably changing the reaction temperature or pressure.

The invention is described concretely with reference to the following Examples, which, however, are not intended to restrict the scope of the invention.

Example 1

(i) Wet grinding treatment of clay

200 ml of distilled water was put into a 500 ml beaker, to which was added 0.1 g of sodium carbonate to prepare an aqueous solution of sodium carbonate. With stirring the aqueous solution, 10 g of Na-montmorillonite (Bengel fromHojun Yoko) was gradually added thereto. After the addition, the resulting clay slurry was transferred into a mortar, and ground in wet for 8 hours in a mill (Yamato Science's Labomill UT-21). To the thus-ground clay slurry, added was distilled water to make 500 ml. The particle size distribution of the clay particles in the resulting colloidal solution was measured with GALAI Production's CIS-1. To measure it, the clay slurry was distilled with water in a ratio of 1/10. As a result, the volume-average particle size of the clay particles was 0.85 μm, and the particles having a particle size of at most 1.5 μm accounted for at least 90 % by weight of all particles.

(ii) Preparation of silane-processed clay

125 ml of the colloidal solution of clay that had been prepared in the previous step was put into a 2 liters three-neck flask, to which was added 825 ml of distilled water. With actively stirring the colloidal solution, 1 ml of phenethylmethyldichlorosilane was added thereto, and then heated up to 100°C. The colloidal solution was further heated at the temperature for 4 hours. The thus-obtained, silane-processed clay slurry was filtered, and the separated silane-processed clay was dried. The yield of the dry product was 2.7 g.

Next, 1.0 g of the dried silane-processed clay was sampled, to which was added 25 ml of a solution of triisobutylaluminium in toluene (0.5 mols/liter), and stirred for 1 hour at 100°C. 200 ml of toluene was added to the resulting slurry and stirred. After having been thus stirred, this was then kept static as it was, and the resulting supernatant was removed through a cannula. Toluene was added to the precipitated solid phase to make a total volume of 50 ml. Thus was prepared a silane-processed clay slurry.

(iii) Preparation of polymerization catalyst

2,6-Diacetylpyridine-bis(2,4-xylylimine)iron dichloride was produced according to the teaching in Chem. Commun., 1998, pp. 849-850. Two ml of a solution of the thus-produced 2,6-diacetylpyridine-bis(2,4-xylylimine)iron dichloride in toluene (2.0 μmols/ml) was added to 5 ml of the silane-processed clay slurry that had been prepared in the previous step, and stirred for 7 hours at room temperature. The catalyst thus prepared contained 0.1 g of clay.

(iv) Polymerization of ethylene

400 ml of toluene and 1.0 mmol of triisobutylaluminium were put into a 1.6 liters autoclave, and heated up to 55°C, to which was added all the catalyst having been prepared in the previous step. This was kept at 55°C for 5 minutes, and then ethylene was continuously introduced thereinto with the ethylene pressure therein being kept 8 kg/$cm^2$G, and polymerized for 30 minutes at a controlled polymerization temperature of 60°C. Next, the autoclave was rapidly cooled and degassed to stop the polymerization therein. 5 minutes after the degassing, the content was taken out, and 192.8 g of a polymerized product was obtained. The content was filtered under pressure at 25°C. As a result, the amount of the oligomer soluble in the polymerization solvent was 161.4 g, and the oligomerization activity of the iron complex used for the polymerization was 2900 kg/g-Fe/hr, in terms of the iron metal in the complex. The oligomer soluble in the polymerization solvent was analyzed through gas chromatography, which confirmed that at least 97 % of oligomers having from 6 to 18 carbon atoms were α-olefins.

Example 2

(i) Preparation of polymerization catalyst

One ml of a solution of 2,6-diacetylpyridine-bis (2, 4-xylylimine) iron dichloride in toluene (2.0 μmols/ml) was added to 2.5 ml of the silane-processed clay slurry that had been prepared in the step (ii) in Example 1, and stirred for 100 hours at room temperature. The catalyst thus prepared contained 0.05 g of clay.

(ii) Polymerization of ethylene

Ethylene was polymerized in the same manner as in the step (iv) in Example 1, except that the polymerization temperature was 80°C and not 60°C herein. As a result, 122.8 g of a polymerized product was obtained. This was processed in the same manner as in Example 1. The amount of the oligomer soluble in the polymerization solvent at 25°C was 98.2 g, and the oligomerization activity of the iron complex was 3520 kg/g-Fe/hr, in terms of the iron metal in the complex. The oligomer was analyzed through gas chromatography, which confirmed that at least 97 % of oligomers having from 6 to 18 carbon atoms were α-olefins.

Comparative Example 1

(i) Polymerization of ethylene

400 ml of toluene and 1.0 mmol of triisobutylaluminium were put into a 1.6 liters autoclave, and heated up to 80°C, to which were added 2.5 ml of the silane-processed clay slurry that had been prepared in Example 1 (ii) and then 1 ml of a solution of 2,6-diacetylpyridine-bis(2,4-xylylimine)iron dichloride in toluene (2.0 μmols/ml) in that order. This was kept at 80°C for 5 minutes, and then ethylene was continuously introduced thereinto with the ethylene pressure therein being kept 8 $kg/cm^2G$, and polymerized for 30 minutes at a controlled polymerization temperature of 80°C. As a result, 10.1 g of a polymerized product was obtained. This was processed in the same manner as in Example 1. The amount of the oligomer soluble in the polymerization solvent at 25°C was 8.1 g, and the oligomerization activity of the iron complex was 290 kg/g-Fe/hr, in terms of the iron metal in the complex.

Example 3

(i) Wet grinding treatment of clay

Clay was processed in the same manner as in Example 1 (i). In this, however, Na-montmorillonite of Kunipia F (from Kunimine Industry) and not Bengel was used, and the wet grinding time was 4 hours and not 8 hours. The particle size distribution of the clay particles in the colloidal solution obtained herein was measured. As a result, the volume-average particle size of the clay particles was 1.03 μm, and the particles having a particle size of at most 1.5 μm accounted for at least 75 % by weight of all particles.

(ii) Preparation of silane-processed clay

A silane-processed clay slurry was prepared in the same manner as in Example 1 (ii).

(iii) Preparation of polymerization catalyst

Two ml of a solution of 2,6-diacetylpyridine-bis (2,4-xylylimine) iron dichloride in toluene (2.0 μmols/ml) was added to 5 ml of the silane-processed clay slurry that had been prepared in the previous step, and stirred for 15 hours at room temperature. The catalyst thus prepared contained 0.1 g of clay.

(iv) Polymerization of ethylene

Ethylene was polymerized in the same manner as in Example 1 (iv), except that the catalyst having been prepared in the previous step was used herein. As a result, 106.1 g of a polymerized product was obtained. This was processed in the same manner as in Example 1. The amount of the oligomer soluble in the polymerization solvent at 25°C was 90.9 g, and the oligomerization activity of the iron complex was 1630 kg/g-Fe/hr, in terms of the iron metal in the complex. The oligomer was analyzed through gas chromatography, which confirmed that at least 97 % of oligomers having from 6 to 18 carbon atoms were α-olefins.

Example 4

(i) Preparation of polymerization catalyst

Two ml of a solution of 2,6-diacetylpyridine-bis(2,4-xylylimine)iron dichloride in toluene (2.0 μmols/ml) was added to 5 ml of the silane-processed clay slurry that had been prepared in Example 3 (ii), and stirred for 15 minutes at room temperature. The catalyst thus prepared contained 0.1 g of clay.

(ii) Polymerization of ethylene

Ethylene was polymerized in the same manner as in Example 1. In this, however, the catalyst that had been prepared in the previous step was used, and the polymerization time was 1 hour. As a result, 58.8 g of a polymerized product was obtained. This was processed in the same manner as in Example 1. The amount of the oligomer soluble in the polymerization solvent at 25°C was 53.5 g, and the oligomerization activity of the iron complex was 480 kg/g-Fe/hr, in terms of the iron metal in the complex. The oligomer was analyzed through gas chromatography, which confirmed that at least 97 % of oligomers having from 6 to 18 carbon atoms were α-olefins.

Comparative Example 2

(i) Polymerization of ethylene

400 ml of toluene and 1.0 mmol of triisobutylaluminium were put into a 1.6 liters autoclave, and heated up to 55°C, to which were added 5 ml of the silane-processed clay slurry that had been prepared in Example 3 (ii) and then 2 ml of a solution of 2,6-diacetylpyridine-bis(2,4-xylylimine)iron dichloride in toluene (2.0 μmols/ml) in that order. In the autoclave, ethylene was polymerized for 1 hour at a polymerization temperature of 60°C and under an ethylene pressure of 8 $kg/cm^2G$. As a result, 8.1 g of a polymerized product was obtained. This was processed in the same manner as in Example 1. The amount of the oligomer soluble in the polymerization solvent at 25°C was 7.9 g, and the oligomerization activity of the iron complex was 140 kg/g-Fe/hr, in terms of the iron metal in the complex.

Example 5

(i) Measurement of particle size distribution

50 mg of a powder of Tsuchiya Kaolin's 250M (this is bentonite for industrial use and its main ingredient is Na-montmorillonite) was suspended in 100 ml of distilled water, and the particle size distribution of the clay particles was measured. As a result, the volume-average particle size of the clay particles was 9.76 μm, and the particles having a particle size of at most 1.5 μm accounted for at least 5 % by weight of all particles.

(ii) Preparation of silane-processed clay

2.5 g of the powder of Tsuchiya Kaolin's 250M was put into a 2 liters three-neck flask, to which was added 1000 ml of distilled water. With actively stirring the resulting slurry, 1 ml of phenethylmethyldichlorosilane was added thereto. Next, this was processed in the same manner as in Example 3 (ii) to prepare a silane-processed clay slurry.

(iii) Preparation of polymerization catalyst

A catalyst containing 0.1 g of clay was prepared in the same manner as in Example 3 (iii). In this, however, the silane-processed clay slurry that had been prepared in the previous step was used, and it was contacted with the solution of 2,6-diacetylpyridine-bis(2,4-xylylimine)iron dichloride in toluene for 15 minutes.

(iv) Polymerization of ethylene

Ethylene was polymerized in the same manner as in Example 3 (iv). As a result, 20.3 g of a polymerized product was obtained. This was processed in the same manner as in Example 1. The amount of the oligomer soluble in the polymerization solvent at 25°C was 19.3 g, and the oligomerization activity of the iron complex was 350 kg/g-Fe/hr, in terms of the iron metal in the complex. The oligomer was analyzed through gas chromatography, which confirmed that at least 97 % of oligomers having from 6 to 18 carbon atoms were α-olefins.

Example 6

(i) Preparation of silane-processed clay

The silane-processed clay slurry that had been prepared in the same manner as in Example 1 (ii) was again washed with toluene. Concretely, 200 ml of toluene was added to the silane-processed clay slurry. After stirred, this was kept static as it was, and the resulting supernatant was removed. After having been washed twice, the silane-processed clay was made to have a volume of 50 ml with toluene.

(ii) Measurement of aluminium concentration in washes

The supernatant having been removed in the toluene washing treatment in Example 1 (ii) (this is the first wash), and the supernatant having been removed in the toluene washing treatment in the previous step (i) herein (this is the second wash) were separately sampled. Each sample was 50 ml. From each sample, toluene was removed under reduced pressure. To each residue, added was 50 ml of an aqueous solution of 1.0 N dilute hydrochloric acid to prepare a uniform solution. The aluminium concentration of the uniform solution was measured by the use of an inductively-coupled plasma emission spectrometer (ICP). As a result, the aluminium concentration in the first wash was 1.2 % by weight (8.4 mmols per gram of the silane-processed clay); and that in the second wash was 0.22 % by weight (0.15 mmols per gram of the silane-processed clay). After having been washed twice, the silane-processed clay was made to have a volume of 50 ml with toluene, and the amount of aluminium still remaining in the supernatant of the clay slurry was measured. It was 0.07 mmols.

(iii) Preparation of polymerization catalyst

A catalyst (containing 0.1 g of clay) was prepared in the same manner as in Example 1 (iii).

(iv) Polymerization of ethylene

Ethylene was polymerized in the same manner as in the step (iv) in Example 1, and 212.1 g of a polymerized product was obtained. This was processed in the same manner as in Example 1. The amount of the oligomer soluble in the polymerization solvent at 25°C was 177.5 g, and the oligomerization activity of the iron complex used for the polymerization was 3200 kg/g-Fe/hr, in terms of the iron metal in the complex. The oligomer was analyzed through gas chromatography, which confirmed that at least 97 % of oligomers having from 6 to 18 carbon atoms were $\alpha$-olefins.

Example 7

(i) Preparation of silane-processed clay

The silane-processed clay slurry that had been prepared in the same manner as in Example 1 (ii) was washed twice more with toluene. Concretely, 200 ml of toluene was added to the silane-processed clay slurry. After stirred, this was kept static as it was, and the resulting supernatant was removed. As repeated twice more herein, the toluene washing treatment for the silane-processed clay slurry was effected three times in all. After having been washed three times, the silane-processed clay was made to have a volume of 50 ml with toluene.

(ii) Measurement of aluminium concentration in wash

50 ml of the supernatant having been removed in the final toluene washing treatment in the previous step (i) (this is the third wash) was sampled, and the aluminium concentration in the sample was measured in the same manner as in Example 6 (ii). The aluminium concentration in the third wash was 46 ppm.

(iii) Preparation of polymerization catalyst

A catalyst (containing 0.1 g of clay) was prepared in the same manner as in Example 1 (iii).

(iv) Polymerization of ethylene

Ethylene was polymerized in the same manner as in the step (iv) in Example 1, and 237.3 g of a polymerized product was obtained. This was processed in the same manner as in Example 1. The amount of the oligomer soluble

in the polymerization solvent at 25°C was 198.5 g, and the oligomerization activity of the iron complex used for the polymerization was 3600 kg/g-Fe/hr, in terms of the iron metal in the complex. The oligomer was analyzed through gas chromatography, which confirmed that at least 97 % of oligomers having from 6 to 18 carbon atoms were α-olefins.

Example 8

(i) Preparation of silane-processed clay

The silane-processed clay slurry that had been prepared in the same manner as in Example 1 (ii) was washed four times more with toluene. Concretely, 200 ml of toluene was added to the silane-processed clay slurry. After stirred, this was kept static as it was, and the resulting supernatant was removed. As repeated four times more herein, the toluene washing treatment for the silane-processed clay slurry was effected five times in all. After having been washed five times, the silane-processed clay was made to have a volume of 50 ml with toluene.

(ii) Measurement of aluminium concentration in wash

50 ml of the supernatant having been removed in the final toluene washing treatment in the previous step (i) (this is the fifth wash) was sampled, and the aluminium concentration in the sample was measured in the same manner as in Example 6 (ii). The aluminium concentration in the fifth wash was lower than the detection limit.

(iii) Preparation of polymerization catalyst

A catalyst (containing 0.1 g of clay) was prepared in the same manner as in Example 1 (iii).

(iv) Polymerization of ethylene

Ethylene was polymerized in the same manner as in the step (iv) in Example 1, and 252.6 g of a polymerized product was obtained. This was processed in the same manner as in Example 1. The amount of the oligomer soluble in the polymerization solvent at 25°C was 206.6 g, and the oligomerization activity of the iron complex used for the polymerization was 3700 kg/g-Fe/hr, in terms of the iron metal in the complex. The oligomer was analyzed through gas chromatography, which confirmed that at least 97 % of oligomers having from 6 to 18 carbon atoms were α-olefins.

INDUSTRIAL APPLICABILITY

As described in detail hereinabove with reference to its embodiments, the catalyst for α-olefin production of the invention has high ethylene oligomerization activity. In addition, in the process of α-olefin production by the use of the catalyst, the amount of side products such as heavy components and waxy components is reduced. Accordingly, the post-treatment of the reaction product produced in the process is easy, and α-olefins are efficiently produced from ethylene at low costs. Further, each component of α-olefins produced has high purity and therefore has high commercial value.

## Claims

1. A catalyst for α-olefin production, which is prepared by contacting (a) any of clay, clay minerals and ion-exchange layer compounds with (b) a transition metal complex of which the center metal is a transition metal of Groups 8 to 10 of the Periodic Table, for a period of at least 10 minutes.

2. The catalyst for α-olefin production as claimed in claim 1, wherein the component (a) has a volume-average particle size of at most 10 μm.

3. The catalyst for α-olefin production as claimed in claim 2, wherein the particles of the component (a) having a volume-average particle size of at most 3.0 μm account for at least 10 % by weight of the total of the component (a).

4. The catalyst for α-olefin production as claimed in any of claims 1 to 3, wherein the component (b) is a transition metal complex of a general formula (1):

$$L^1L^2L^3MX^1{}_mY^1{}_n \quad (1)$$

where M represents a transition metal of Groups 8 to 10 of the Periodic Table; at least one of $L^1$ to $L^3$ represents a ligand capable of bonding to the transition metal via a hetero atom, and these may be bonded to each other to form a ring; $X^1$ and $Y^1$ each represent a covalent-bonding or ionic-bonding ligand, and they may be the same or different; m and n each indicate 0 or a positive integer, and the sum of m and n is 0, 1, 2 or 3, depending on the valency of M.

**5.** The catalyst for α-olefin production as claimed in claim 4, wherein all of $L^1$ to $L^3$ in formula (1) are ligands capable of bonding to the transition metal via a hetero atom.

**6.** The catalyst for α-olefin production as claimed in any of claim 1 to 5, wherein the component (b) is a transition metal complex having a polydentate ligand that bonds to the transition metal via a hetero atom.

**7.** The catalyst for α-olefin production as claimed in any of claims 1 to 6, wherein the component (a) is an organosilane compound-processed clay, clay mineral or ion-exchange layer compound.

**8.** The catalyst for α-olefin production as claimed in claim 7, wherein the organosilane compound is represented by a general formula:

$$R_nSiX_{4-n}$$

where R represents a substituent in which the atom directly bonding to the silicon atom is a carbon, silicon or hydrogen atom; X represents a substituent in which the atom directly bonding to the silicon atom is a halogen, oxygen or nitrogen atom; a plurality of R's and X's, if any, may be the same or different; n indicates an integer of from 1 to 3.

**9.** A method for producing α-olefins, which comprises oligomerizing ethylene in the presence of the catalyst for α-olefin production of any of claims 1 to 8.

**10.** A catalyst for α-olefin production, which is prepared by contacting (a') any of clay, clay minerals or ion-exchange layer compounds having been processed with an alkylating agent, with (b) a transition metal complex of which the center metal is a transition metal of Groups 8 to 10 of the Periodic Table, wherein the component (a') is such that its suspension formed by adding an inert medium thereto releases at most 1 mmols, per gram of the component (a'), of the alkylating agent in the medium.

**11.** The catalyst for α-olefin production as claimed in claim 10, wherein the component (a') has a volume-average particle size of at most 10 μm.

**12.** The catalyst for α-olefin production as claimed in claim 11, wherein the particles of the component (a') having a volume-average particle size of at most 3.0 μm account for at least 10 % by weight of the total of the component (a').

**13.** The catalyst for α-olefin production as claimed in any of claims 10 to 12, wherein the component (b) is a transition metal complex of above formula (1).

**14.** The catalyst for α-olefin production as claimed in claim 13, wherein all of $L^1$ to $L^3$ in formula (1) are ligands capable of bonding to the transition metal via a hetero atom.

**15.** The catalyst for α-olefin production as claimed in any of claims 10 to 14, wherein the component (b) is a transition metal complex having a polydentate ligand that bonds to the transition metal via a hetero atom.

**16.** The catalyst for α-olefin production as claimed in any of claims 10 to 15, wherein the component (a') is any of clay, clay minerals or ion-exchange layer compounds having been processed with an organosilane compound and then with an alkylating agent.

**17.** The catalyst for α-olefin production as claimed in claim 16, wherein the organosilane compound is represented by a general formula:

$$R_nSiX_{4-n}$$

where R, X and n have the same meanings as above.

**18.** A method for producing α-olefins, which comprises oligomerizing ethylene in the presence of the catalyst for α-olefin production of any of claims 10 to 17.

## INTERNATIONAL SEARCH REPORT

International application No.
PCT/JP00/03724

A. CLASSIFICATION OF SUBJECT MATTER
Int.Cl[7] B01J 31/26, C07C2/32, C07C11/02, C08F4/80

According to International Patent Classification (IPC) or to both national classification and IPC

B. FIELDS SEARCHED

Minimum documentation searched (classification system followed by classification symbols)
Int.Cl[7] B01J 21/00-37/36, C07C2/32, C07C11/02, C08F4/80

Documentation searched other than minimum documentation to the extent that such documents are included in the fields searched

| | | | |
|---|---|---|---|
| Jitsuyo Shinan Koho | 1926-1996 | Toroku Jitsuyo Shinan Koho | 1994-2000 |
| Kokai Jitsuyo Shinan Koho | 1971-2000 | Jitsuyo Shinan Toroku Koho | 1996-2000 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| PA | JP, 11-269224, A (Idemitsu Petrochem Co., Ltd.), 05 October, 1999 (05.10.99) & EP, 985685, A | 1-18 |
| A | JP, 10-81635, A (Idemitsu Kosan Co., Ltd.), 31 March, 1998 (31.03.98), (Family: none) | 1-18 |
| A | JP, 08-332390, A (NIPPON OIL COMPANY, LTD.), 17 December, 1996 (17.12.96), & WO, 96027400, A & EP, 758563, A & US, 5744678, A | 1-18 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>12 September, 2000 (12.09.00) | Date of mailing of the international search report<br>19 September 2000 (19.09.00) |
| Name and mailing address of the ISA/<br>Japanese Patent Office<br>Facsimile No. | Authorized officer<br><br>Telephone No. |

Form PCT/ISA/210 (second sheet) (July 1992)